Europäisches Patentamt

European Patent Office

Office européen des brevets

(11) Publication number: **0 401 648**
**A1**

(12) **EUROPEAN PATENT APPLICATION**

(21) Application number: **90110186.5**

(22) Date of filing: **29.05.90**

(51) Int. Cl.⁵: **G01N 33/24, G01N 31/00**

(30) Priority: **05.06.89 IT 2078989**

(43) Date of publication of application:
**12.12.90 Bulletin 90/50**

(84) Designated Contracting States:
**DE DK ES FR GB IT NL SE**

(71) Applicant: **CARLO ERBA STRUMENTAZIONE S.p.A.**
**Casella Postale Aperta**
**I-20090 Rodano Milano(IT)**

(72) Inventor: **Colombo, Bruno**
**Via Perego 2**
**I-20093 Cologno Monzese (Milano)(IT)**
Inventor: **Baccanti, Marco**
**Via Stradella 8**
**I-20129 Milano(IT)**

(74) Representative: **Marietti, Giuseppe**
**CENTRO DI CONSULENZA IN PROPRIETA'**
**INDUSTRIALE Viale Caldara, 38**
**I-20122 Milano(IT)**

(54) **Process and equipment for the determination of organic carbon content in complex matrixes.**

(57) The determination of organic carbon in complex matrixes such as soils, geological samples, cements and the like is carried out by weighing a predetermined amount of a sample of said matrices in a capsule of Ag, Cu or their alloys, extra-acidifying said sample, bringing to dryness the thus acidified sample at a temperature below 100 degrees C and by submitting the dried sample to an elemental analysis by combustion and mesurement of produced $CO_2$.

Fig.1

# PROCESS AND EQUIPMENT FOR THE DETERMINATION OF ORGANIC CARBON CONTENT IN COMPLEX MATRIXES

The present invention concerns a process and an equipment for the determination of the percentage of organic carbon in complex matrixes, such as matrixes rich in carbonates as for example soils, sediments, residues from ashing furnaces and cements.

The determination of the total organic component in complex matrixes such as those mentioned above has proved to be extremely important in analytical chemistry applied to agronomy, geology or to the control and handling of industrial processes such as for example cement production.

The classical method for the determination of organic carbon in a matrix envisages oxidation with potassium dichromate and subsequent titration or spectrophotometric measurement. Such a method, known as UNICHIM method (WALKLEY-BLACK), though being precise and reliable, has the disadvantage of requiring relatively long analytical times, and in any case such as to reduce the number of samples which can be daily analysed. Furthermore the UNICHIM (WALKLEY-BLACK) method involves high costs and problems concerning the disposal of residues from the chrome solution used during the analysis.

To overcome these problems two different alternative methods were proposed in the past designed to automatise the sample analysis and to reduce relevant costs.

The first of said methods proposes to eliminate carbonates possibly present in the sample to be analysed by means of acidification of the sample itself and its subsequent filtration and drying; the dried sample is then sent to automatic elemental analysis by combustion at 1000 degrees C and subsequent measurement of produced carbon dioxide.

This method, however, has two main disadvantages. First of all the experimental result obtained does not take into consideration possible organic components soluble in the acid solution (such as some wet acids) which are then lost therewith following filtration. Moreover, the experimental datum is not homogeneous with the one relating to total carbon (obtained on the untreated sample) in that it refers to different conditions of relative humidity.

The second process exploits the relative thermal stability of the most common carbonates performing combustion at a temperature of approximately 500 degrees C, which allows to oxidate to carbon dioxide the organic matrix present in the sample, leaving carbonates substantially untouched, which are subsequently decomposed at temperatures exceeding 900 degrees C. It has however been proved that said process is hardly reproducible, in that the different composition of the matrix can show in turn either organic components, the complete combustion of which requires temperatures exceeding 500 degrees, or the presence of carbonates, such as magnesium carbonate, with lower thermal stability and which decompose at temperatures around 500 degrees C.

There is therefore the need of a process for the determination of organic carbon in complex matrixes rich in carbonates, which can have a precision and reproducibility at least corresponding to those obtainable by the UNICHIM (WALKLEY-BLACK) method and which is at the same time easily performable, quick and economic.

Object of the present invention is to provide a process and an equipment to carry out the determination of organic carbon content in complex matrixes rich in carbonates, in an easy, quick and economic way and with high reproducibility.

Said object is achieved by means of the process and the equipment according to the present invention which allow the determination of organic carbon content and, indirectly, of inorganic carbon, to be performed with extreme easiness, reproducibility and quickness.

More in particular the present invention concerns a process for the determination of organic carbon content in complex matrixes, characterized by the fact of comprising the stages of:

weighing a predetermined amount of sample to be analysed in a capsule or analytical container;

acidifying said sample in said capsule by means of an acid solution in stechiometrically higher quantity than that necessary for the reaction of possible carbonates present in the matrix;

heating the acidified sample in the capsule at a temperature below 100 degrees C approximately up to its substantial dryness; and

carrying out an elemental analysis by dynamic and quantitative flash combustion of the sample thus dried in order to determine its organic carbon content directly in said analytical capsule.

The invention furthermore concerns an equipment for the determination of organic carbon content in complex matrixes, of the type comprising means for weighing, acidifying and subsequently bringing to dryness a sample of said matrixes, as well as means for dynamic and quantitative flash combustion of said sample and for the measurement of carbon dioxide thus produced, characterized in that it comprises capsules or analytical containers made of silver, copper or their alloys.

The invention will be now further described with reference to the accompanying example and drawings, where:

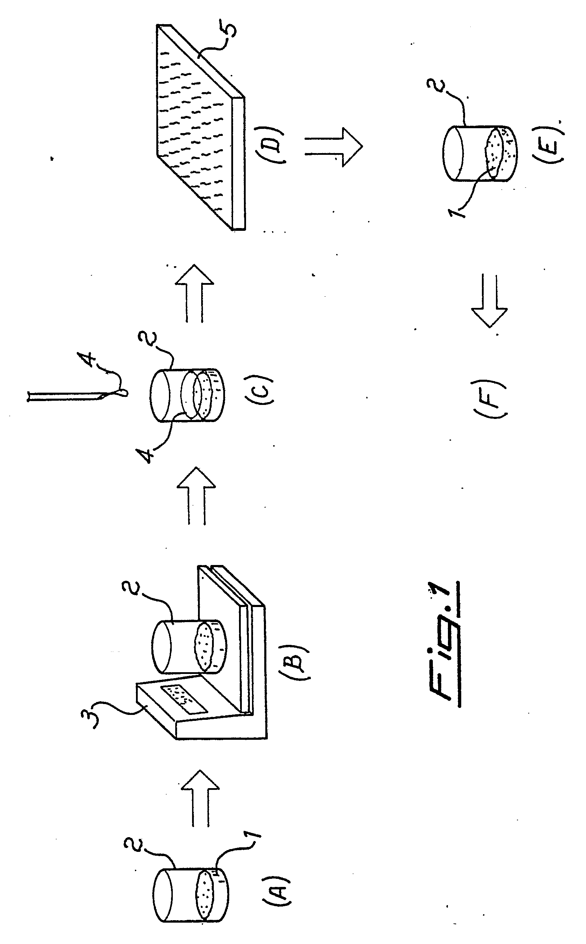

figure 1 is a schematic diagram of the different steps of the process according to the invention.

With reference to figure 1, the process according to the invention foresees to place first of all a predetermined amount of sample 1 of the matrix to be analysed in a capsule or analytical container (stage A) and to exactly weigh said amount on a balance 3 (stage B). The sample thus weighed and contained in the capsule 2 is additioned with an aqueous solution 4 of a mineral acid at such a concentration as to provide a stechiometrically higher quantity of acid than the one necessary for the reaction of all possible carbonates present in the matrix (stage C). In particular the aqueous acid solution 4 must wet all the sample present within the capsule 2.

Preferably the acid solution used consists of a solution of 37% hydrochloric acid diluted in distilled water in a 1:1 ratio. The capsules used with said acid are preferably made of silver or copper or their alloys.

As it can be noticed in figure 1, the sample amount placed inside the capsule 2 is laid in a way as to form a layer covering the bottom of the capsule in a substantially uniform way and presenting a height not exceeding approximately one third of the height of the capsule itself. This operative procedure allows all the sample to be wetted and sample losses to be avoided during sample acidification and carbon dioxide development by reaction of carbonates.

It has been experimentally proved that for a usual quantity of sample, i.e. in the order of 10-20 mg of substance, approximately 6-8 microliters of the previously mentioned 18% HCl solution are sufficient. For said amounts the use of capsules having circular section, diameter ranging between about 5 and 7 mm and height ranging between about 7 and 9 mm has proved to be particularly advantgeous. In said capsules the preferential average height of the sample layer is approximately 2 mm.

After the sample acidification and the carbonates reaction, the capsule together with the wet and acidified sample thus obtained is put in presence of heating means such as for instance a heating plate 5 (stage D), which perform evaporation of the water contained in the capsule and deriving from the hydrochlorid acid solution. In order to avoid the loss of volatile organic substances, the heating temperature of the capsule 2 and the sample 1 is lower than approximately 100 degrees C and preferably ranges between 80 and 100 degrees C.

The sample complete anhydrification is not critical for carrying out the process, but it is however preferred to bring the sample to substantial dryness.

In this case as well it has been experimentally proved that, though it is possible to use lower temperatures, temperatures coming within said range substantially allow to eliminate all water and humidity present in the sample, including that generally kept by calcium salts formed, in a sufficiently short period of time.

Once evaporation of humidity present in the sample is carried out and after the sample has been brought to substantial dryness (stage E), the capsule containing the sample is then sent to an elemental analysis (stage F) by dynamic and quantitative flash combustion, with subsequent measurement of produced carbon dioxide, namely an alemental analysis according to a technique of traditional type.

It is here pointed out that all operations are performed on the sample contained in the capsule without any handling of it, as it occures, on the contrary, in case of acidification and filtration of the sample according to the known technique. The experimental datum obtained by the process according to the invention is therefore homogeneous with that relating to the percentage of total carbon obtained by combustion and measurement of produced $CO_2$ and at the same time it takes into account soluble organic species.

Now an example of analysis of a soil sample carried out by the process and the equipment according to the invention is hereunder reported.

## EXAMPLE 1

Approximately 18 mg of a soil sample are weighed in an Ag capsule with 6.5 mm diameter and 8 mm height, treated with approx. 7 microliters of 18% HCl and then dried placing the capsule on a plate heated at 80 degrees C for 5 minutes.

The capsule containing the treted sample was then sent to an Elemental Analyser CARLO ERBA STRUMENTAZIONE type NA 1500.

The following tables summarize the results obtained performing the analysis of a series of samples of soils and sediments both by the UNICHIM (WALKLEY-BLACK) method and the method according to the invention as described in the abovereported example. As it can be noted, the results obtained by the process according to the invention are perfectly compatible with those obtained according to the UNICHIM (WALKLEY-BLACK) method and show excellent coefficients of variation. Organic carbon analysis by combustion and measurement of carbon dioxide produced was carried out using a CARLO ERBA Analyser series NA-1500, but it can obviously be performed by means of any other type of elemental analyser without any substantial variation in the hereunder reported results.

TABLE I

| TOTAL ORGANIC CARBON DETERMINATION AND COMPARISON WITH UNICHIM (WALKLEY-BLACK) METHOD | | |
|---|---|---|
| ORG.C. Walkley-Black (UNICHIM) | ORG.C. (invention) | TOTAL C. (elemental analyser) |
| 1.56 | 1.52-1.54-1.57 | 8.42 |
| 2.10 | 2.19-2.14-2.16 | 4.38 |
| 3.28 | 3.11-3.10-3.14 | 4.91 |
| 0.96 | 1.04-1.04-1.06 | 2.34 |
| 1.17 | 1.47-1.44-1.42 | 4.85 |
| | 1.44-1.44-1.42 | |
| 0.97 | 1.00-1.01-1.01 | 2.52 |

TABLE II

| REPRODUCIBILITY | |
|---|---|
| UNICHIM (WALKLEY-BLACK) | INVENTION |
| 0.65 | 0.69 |
| 0.70 | 0.73 |
| 0.69 | 0.74 |
| 0.65 | 0.70 |
| 0.70 | 0.70 |
| s = 0.0259 | s = 0.0217 |
| V.C. = 3.82% | V.C. = 3.04% |

The two analyses for organic carbon and total carbon may be performed on the same instrument for elemental analysis (for example a Carlo Erba Strumentazioone elemental analyser), obviously not performing stages A-E in case of total carbon determination. In this way it is possible to obtain indirectly, by difference, the value of the inorganic carbon content as well.

Furthermore, the in situ treatment of the sample proposed by the process according to the invention allows the determination of total organic carbon to be obtained without loss of any volatile compounds or soluble compounds in acid aqueous solutions.

From what reported hereinabove and considering that the total time of an analytical cycle according to the invention, relating to the determination of organic carbon percentage by weight, is on the average 5/6 minutes approximately, it will become obvious that the process according to the invention allows to determine in a reliable, simple, economic and quick way the organic carbon content and, indirectly, the inorganic carbon content, of whatever complex matrix containing carbonates.

## Claims

1. Process for the determination of organic carbon content in complex matrixes, characterized by the fact of comprising the stages of :

weighing a predetermined amount of sample to be analysed in a capsule or analytical container;

acidifying said sample in said capsule with an acid solution in stechiometrically higher quantity than that necessary for the reaction of all possible carbonates present in the matrix;

heating the acidified sample in the capsule at a temperature below 100 degrees C approx., up to its substantial dryness; and

performing an elemental analysis by dynamic and quantitative flash combustion of the dried sample to determine its organic carbon content directly in said analytical capsule.

2. Process according to claim 1, characterized in that said sample is laid within said analytical capsule in a substantially uniform layer with a height less than one third of the height of the capsule itself.

3. Process according to claim 1 or 2, characterized in that said sample is acidified by aqueous hydrochloric acid and in that capsules of silver, copper or their alloys are used.

4. Process according to claim 3, characterized in that a 1:1 solution of 37% hydrochloric acid and distilled water is used.

5. Process according to one of the claims from 1 to 3, characterized in that said sample is heated at a temperature ranging between 80 and 100 degrees C.

6. Equipment for the determination of organic carbon content in complex matrixes, of the type comprising means for weighing, acidifying and subsequently bringing to dryness a sample of said matrixes and means for dynamic and quantitative flash combustion of said sample and for the measurement of produced carbon dioxide, characterized in that it comprises analytical capsules or containers made of silver, copper of their alloys.

7. Equipment according to claim 6, characterized in that said capsules are capsules with circular section with diameter ranging between approximately 5 and 7 mm and height ranging between approximately 7 and 9 mm.

_Fig.1_

EP 0 401 648 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | BE-A-852335 (LABOFINA S.)<br>* claims 1-3; figure 1 *<br>--- | 1, 6 | G01N33/24<br>G01N31/00 |
| A | GB-A-2066462 (INSTITUT FRANCAIS DU PETROL)<br>* abstract; figure 1 *<br>--- | 1, 6 | |
| A | US-A-3854881 (COHEN A.)<br>* abstract; figure 1 *<br>----- | 1, 3, 6 | |

| | |
|---|---|
| | TECHNICAL FIELDS SEARCHED (Int. Cl.5)<br><br>G01N |

The present search report has been drawn up for all claims

| Place of search | Date of completion of the search | Examiner |
|---|---|---|
| THE HAGUE | 03 SEPTEMBER 1990 | BAROCCI S. |